## Europäisches Patentamt

## European Patent Office

## Office européen des brevets

(11) Veröffentlichungsnummer: **0 093 436**
**B1**

(12) **EUROPÄISCHE PATENTSCHRIFT**

(45) Veröffentlichungstag der Patentschrift:
05.11.86

(21) Anmeldenummer: 83104243.7

(22) Anmeldetag: 29.04.83

(51) Int. Cl.⁴: **C 12 N 15/00, C 12 N 5/00,**
**C 12 P 1/00, C 12 P 21/00 //**
**C12R1/91**

(54) Verfahren zur Herstellung permanenter tierischer und humaner Zellinien und deren Verwendung.

(30) Priorität: 04.05.82 DE 3216650
09.12.82 DE 3245665

(43) Veröffentlichungstag der Anmeldung:
09.11.83 Patentblatt 83/45

(45) Bekanntmachung des Hinweises auf die Patenterteilung:
05.11.86 Patentblatt 86/45

(84) Benannte Vertragsstaaten:
AT BE CH DE FR GB IT LI LU NL SE

(56) Entgegenhaltungen:
BIOLOGICAL ABSTRACTS, Band 66, Nr. 5, 1.
September 1978, Seite 2666, Zusammenfassung Nr.
27083, Philadelphia, US E. MUNRO u.a.:
"Cytoplasmic transfer of a determinant for
chloramphenicol resistance between mammalian
cell lines"
CHEMICAL ABSTRACTS, Band 86, Nr. 17, 25. April
1977, Seite 360, Nr. 118951q, Columbus, Ohio, US A.
WACKER u.a.: "Polyethylene glycol-induced fusion
of mammalian cells with cytoplasts in suspension"
CHEMICAL ABSTRACTS, Band 82, Nr. 23, 9. Juni
1975, Seite 252, Nr. 151810h, Columbus, Ohio, US
M.H. WIGLER u.a.: "Preparative metod for
obtaining enucleated mammalian cells"
CHEMICAL ABSTRACTS, Band 86, Nr. 21, 23. Mai
1977, Seite 202, Nr. 152366n, Columbus, Ohio, US M.
JETT u.a.: "Isolation and characterization of
plasma membranes and intact nuclei from
lymphoid cells"
MEDICAL LABORATORY SCIENCES, Band 36, 1979,
Seiten 329-338, The Institute of Medical Laboratory
Sciences A.D. BLANN: "Cell hybrids: an important
new source of antibody production"

(73) Patentinhaber: BOEHRINGER MANNHEIM GMBH,
Patentabteilung, Abt. E Sandhofer Strasse 112-
132 Postfach 31 01 20, D-6800 Mannheim 31
Waldhof (DE)

(72) Erfinder: Jungfer, Herbert, Dr. med., Beringerweg
10, D-8132 Tutzing (DE)
Erfinder: Barchet, Heinrich, Bräuhausstrasse 3,
D-8132 Tutzing (DE)
Erfinder: Albert, Winfried, Dr. phil., Moosstrasse
10, D-8121 Pähl (DE)

(74) Vertreter: Weickmann, Heinrich, Dipl.- Ing.,
Patentanwälte Dipl.- Ing. H.Weickmann Dipl.-
Phys.Dr. K.Fincke Dipl.- Ing. F.A.Weickmann
Dipl.- Chem. B. Huber Dr.- Ing. H. Liska Dipl.-
Phys.Dr. J. Prechtel Postfach 860820, D-8000
München 86 (DE)

(56) Entgegenhaltungen: (Fortsetzung)
Immunology, John Wiley and Sons, pages 43 and 47
A Dictionary of Immunology, Blackwell Scientific
publications; Herbert W.J. and P.C. Wilkinson, 1977
2nd edition, page 97
Nucleic Acids Research, Vol. 5, no. 7, July 1978,
p.2313-19
Sciences, Vol. 206, 19 October 1979, P. 337-344
Proceedings National Academy of Sciences USA,
Vol. 78, N. 3, March 1981, pp. 1411-15
Cell and Tissue culture- John Paul, 5th Edition,
Churchill Livingstone, pp. 82-83
Molecular Biology of "The Cell", Garland
Publishing, Inc., Bruce Alberts et al., p. 163

## Beschreibung

Die Erfindung betrifft ein Verfahren zur Herstellung von permanent züchtbaren tierischen und humanen Zellinien und die Verwendung so erhaltener Zellinien zur Gewinnung von Zellprodukten. Seit langem bemüht man sich, sowohl aus wissenschaftlichen als auch aus praktischen Gründen menschliche und tierische Zellen unabhängig vom normalen tierischen oder menschlichen Gewebe permanent zu züchten. Bisher ist dies nicht befriedigend gelungen und nur in wenigen Sonderfällen konnte eine permanente Züchtbarkeit bei bestimmten Blutzellen erzielt werden.

Es ist bekannt, zur Herstellung monoklonaler Antikörper mit definierter Antigen-Bindungsspezifität die sogenannte Hybridoma-Technik anzuwenden. Mit diesem von Köhler und Milstein (Continuous culture of fused cells secreting antibody of predefined specificity, Nature 256, 495-497 (1975)) entwickelten Verfahren kann eine einzelne, Antikörper (AK) bildende Zelle potentiell "unsterblich" gemacht und beliebig vermehrt werden. Durch Fusion der AK-bildenden Zellen (B-Lymphocyt) mit einer maligne entarteten Zelle (Myelom) können Zellhybride geschaffen werden, die die Eigenschaften beider Elternteile in sich vereinen: Die Fähigkeit, Antikörper zu produzieren und die Fähigkeit zu permanentem Wachstum. Das neue Wort "Hybridoma" wurde durch Fusion von Hybrid-Zelle und Myeloma gebildet.

Zum leichteren Verständnis der Besonderheiten dieser Technik sollen einige Grundlagen der Struktur und Synthese von Antikörpern (Immunglobuline, Ig) dargestellt werden. Ein Ig-Molekül setzt sich aus zwei identischen Leicht-(L) und zwei identischen Schwer-(H)-Ketten zusammen. Jede H- und L-Kette ist in genetisch und funktionell unterschiedliche Abschnitte aufgeteilt. Die Antigen-Bindungsstellen des Antikörpers (englisch: combining sites) sind in den sog. variablen Regionen ausgebildet, die einen hohen Grad an Sequenz-Heterogenität aufweisen. Die vielfältigen Aminosäuren-Austausche erzeugen ein großes Repertoire von dreidimensionalen Strukturen, die in ihrer Form komplementär zu einer großen Anzahl von Antigenen sind. Man schätzt, daß ein Säuger zwischen $10^6$ und $10^7$ verschiedene Antigen-Bindungsstellen ausbilden kann.

Antikörper sind das Synthese-Produkt von B-Lymphocyten. Während der ontogenetischen Entwicklung einer B-Zelle aus einer Stammzelle wird eins der vielen verfügbaren Variablen-Region-Gene mit einem der vergleichsweise wenigen Konstant-Region-Genen kombiniert, und zwar sowohl für die L- wie die H-Kette. Sobald die Gen-Assoziierung erfolgt ist, ist die betreffende B-Zelle darauf festgelegt, nur einen einzigen Typ von Antikörper-Molekül zu bilden, und diese Festlegung vererbt sie ihren Tochterzellen. Ohne Antigen-Stimulus verharrt die B-Zelle in einem Ruhezustand, ohne zu proliferieren. Sie produziert und sezerniert nur wenig Immunglobulin, trägt aber in ihrer Zellmembran fest verankert Antikörper, die exakt die gleiche Antigen-Bindungsstelle haben wie der sezernierte Antikörper. Wenn ein Antigen in den Organismus eindringt, wird es in einer Serie von komplexen zellulären Interaktionen den B-Zellen präsentiert.

Die B-Zelle, deren Membran-Immunglobulin mit dem Antigen spezifisch reagiert, wird dazu gebracht, sich zu teilen und einen Klon von Tochterzellen zu bilden, die zu Antikörper-produzierenden Zellen (Plasmazellen) differenzieren. Da ein B-Zellklon Antikörper mit identischer Struktur und mit identischen Antigen-Bindungsstellen bildet, wird das Produkt eines solchen Klons "monoklonaler Antikörper" genannt. Komplex aufgebaute Antigene wie Proteine, Mikroorganismen oder Zellen enthalten viele verschiedene, Antigen-wirksame Stellen (Determinanten, Epitope) und folgerichtig werden viele verschiedene B-Zellen stimuliert, sich zu teilen und Klone auszubilden. Deshalb wird eine große Vielzahl von Antikörpern gebildet, die sich hinsichtlich ihrer Größe, Ladung, Spezifität und Affinität unterscheiden und vereint im Immunserum erscheinen. Aber auch die gegen eine einzige Determinante gerichtete Immunantwort ist in aller Regel polyklonal. Es ist bekannt, daß Mäuse bis zu $10^3$ verschiedene Antikörper gegen ein einfaches Hapten, d. h. eine isolierte Determinante, bilden können. Diese Tatsachen verdeutlichen, daß es äußerst schwierig, wenn nicht unmöglich ist, in reproduzierbarer Weise Antisera gegen ein bestimmtes Antigen zu erzeugen. Viele Jahre wurde deshalb nach einem Verfahren gesucht, das gestattet, vereinzelte B-Zellen klonal zu expandieren, um auf diese Weise zu homogenen, monoklonalen Antikörpern zu gelangen. Die natürlichen Vorbilder waren die Myelome bzw. Plasmacytome, die als maligne Erkrankungen seit langem in Mäusen, Ratten und Menschen bekannt waren. Ein Myelom entsteht, wenn eine B-Zelle maligne entartet und ungehemmt proliferiert, wobei der Klon von Tochterzellen große Mengen an homogenen Antikörpern produziert. Myelome können in gewissen Inzucht-Mausstämmen durch chemische Manipulation induziert werden. Alle Versuche, durch Kombination von Hyperimmunisierung und Myelom-Induktion zu monoklonalen Antikörpern mit bekannter Antigen-Bindungsspezifität zu kommen, sind jedoch erfolglos geblieben. Immerhin führten diese Bemühungen zu Myelom-Zellinien, die in vitro kultivierbar waren und zur Grundlage der Hybridoma-Technologie geworden sind. Die grundlegende Idee von Milstein und Köhler war, eine Hybridzelle durch Fusion zu normalen B-Zellen aus immunisierten Tieren mit einer kulturfähigen und permanent wachsenden Myelomzelle zu erzeugen.

In ihren ersten Versuchen fusionierten sie die Myelomzellen mit den Lymphocyten aus der Milz einer Maus, die mit Schaf-Erythrocyten immunisiert worden war. Sie erhielten 10 lebensfähige Hybride, von denen zwei Antikörper mit Spezifität gegen Schaf-Erythrocyten bildeten. Die Antikörper-produzierenden Hybride ähnelten den Myelomzellen insoweit, als daß sie kontinuierlich in Kultur wuchsen und Tumoren bildeten, wenn sie syngeneischen Mäusen implantiert wurden. Von großer praktischer Bedeutung war weiterhin, daß die Hybridzellen wie Myelomzellen in Flüssigstickstoff eingelagert und über lange Zeiträume lebensfähig konserviert werden konnten.

## Technik der Hybridoma-Herstellung

Mäuse werden wie bei der konventionellen Antiserum-Herstellung mit Antigen immunisiert, in der Regel wiederholt mit mehrwöchigen Unterbrechungen. Unmittelbar vor der Fusion wird die Maus getötet und ihre Milz unter aseptischen Bedingungen exzidiert. Der Milzsack wird aufgeschnitten und die Milzpulpa vorsichtig herausgedrückt. Die Milzlymphocyten (ca. $10^8$ Zellen) werden in Zellkulturmedium suspendiert und mit Myelomzellen im Verhältnis 1:1 bis 10:1 vermischt. Die Zellmischung wird durch Zentrifugation dicht auf den Boden eines Röhrchens gepackt und nach Entfernung des flüssigen Überstandes mit dem Fusionsmedium (30 bis 50 %ige Polyäthylen-Glycol-Lösung oder suspendiertes, inaktiviertes Sendai-Virus) behandelt. Nach Auswaschen des Fusionsmediums wird die Zellmischung in einer Zelldichte von ca. $10^6$ Zellen pro ml in sterile Kulturgefäße (Tüpfelplatten) überführt und in einem $CO_2$-begastem Inkubator kultiviert. 2 bis 4 Wochen nach Fusion wird das Wachstum von Hybridoma-Klonen mikroskopisch sichtbar. Ab diesem Zeitpunkt kann der Kulturüberstand auf Vorhandensein von Antikörpern mit der gesuchten Spezifität untersucht werden. Dazu sind Analysenverfahren notwendig, die Antikörper im Sub-Mikrogramm-Bereich nachweisen können (RIA, ELISA, Immunofluoreszenz). Die Zellen aus positiven Teilkulturen werden sodann kloniert, d. h. Einzel-Zellkulturen angelegt. Isolierte Klone, die den "richtigen" Antikörper bilden, werden expandiert und zur Tumorinduktion in die Bauchhöhle von Pristan-vorbehandelten syngeneischen Mäusen (in der Regel Balb/c-Inzucht-Mäuse) gespritzt. 6 bis 20 Tage nach der Inokulierung können bei Angehen des Tumors homogene Antikörper aus dem Blut oder vorzugsweise aus der Bauchhöhle (Ascites) gewonnen werden (mit einer Ausbeute von 50 bis 150 mg monoklonaler Antikörper pro Maus).

Nach der Fusion liegt ein sehr heterogenes Gemisch aus Hybriden und nichtfusionierten Zellen vor. Bei Einsatz von $10^8$ Maus-Milzzellen kann man mit max. $10^3$ lebensfähigen Hybridoma-Zellen rechnen. Da die Hybridzellen eine gewisse Anlaufzeit brauchen, bevor sie mit der Proliferation beginnen können, die nicht-fusionierten Myelomzellen aber sofort weiterwachsen, muß ein Selektionsverfahren das Überleben der wenigen Hybridoma sichern. Das Standard-Selektionsverfahren in der Hybridoma-Technik basiert auf dem sogenannten HAT-Selektionsmedium (Littlefield, J.W: Selection of hybrids from matings of fibroblasts in vitro and their presumed recombinants. Science 145, 709-710 (1964)). A steht für Aminopterin, einem Folsäure-Antagonisten, der den Hauptweg der DNS-Synthese blockiert. Normale Zellen können den Aminopterin-Block mit Hilfe von Thymidin-Kinase (TK) und Hypoxanthin-Guanin-Phosphoribosyl-Transferase (HGPRT) umgehen, sofern ihnen im Kulturmedium Thymidin (T) und Hypoxanthin (H) angeboten werden. Fehlt einer Zelle eines der beiden Enzyme, dann ist sie in HAT-Medium nicht überlebensfähig. Für die Hybridoma-Entwicklung werden deshalb Mutanten von Myelomzellen verwendet, welche TK- oder HGPRT-defizient sind. Diese Zellen sind in HAT-Medium nur dann lebensfähig, wenn sie mit einer normalen Zelle fusioniert sind, die mit ihrem Genpool das fehlende Enzym in die Hybridzelle einbringt. Die nicht-fusionierten Lymphocyten der Milz haben in Kultur eine natürlich begrenzte Lebensdauer und stellen deshalb keine Bedrohung der Hybridoma dar.

Bei der Hybridoma-Herstellung treten nun folgende Probleme auf:

1. HAT-Medium-Selektion

Die selektive Unterdrückung des Wachstums von nicht-fusionierten Myelomzellen ist, wie oben dargestellt, eine essentielle Voraussetzung für die Erzeugung von Hybridoma-Klonen. Die HAT-Selektion ist aber auch für normale, nichtdefiziente Zellen ein äußerst unphysiologisches Verfahren, das die Teilungs- und Überlebensfähigkeit der Zellen beeinträchtigt. Besonders bei menschlichen Lymphocyten ist es außerordentlich schwierig, die Komponenten des HAT-Mediums konzentrationsmäßig so einzustellen, daß HGPRT-negative Zellen zuverlässig abgetötet werden, HGPRT-positive Zellen dagegen überleben können.

Das Mißverhältnis zwischen der Zahl an eingesetzten Lymphozyten und Myelomzellen einerseits und der Ausbeute an vermehrungsfähigen Hybriden andererseits veranschaulichen folgende Zahlen: Bei Eingabe von $10^8$ Maus-Lymphocyten in einen typischen Fusionsansatz gelten 500 Hybridoma-Klone allgemein als sehr gutes Ergebnis. Da in der Milz einer Maus - auch wenn sie wiederholt (hyper-)immunisiert worden ist - nur eine von $10^4$ bis $10^3$ Zellen Antikörper gegen das Immunogen bildet (wie mittels Jerne-Plaque-Technik Jerne, N.V. and NORDIN, A.A.: Science 140, 405 (1963) nachgewiesen worden ist), müßten bei rein zufallsbedingter Hybridoma-Bildung $10^3$ bis $10^4$ Klone aufgezogen werden, um auch nur einen Klon mit gewünschter Antikörper-Spezifität erwarten zu können. Bei der Herstellung menschlicher Hybridoma ist das Mißverhältnis noch krasser: Als gutes Ergebnis gilt, wenn pro Fusion 4 bis 10 Hybrid-Klone erhalten werden.

2. Chromosomen-Verluste

Nach einer erfolgreichen Fusion muß die neu entstandene Hybridzelle mit etwa der doppelten, von der Natur ursprünglich vorgesehenen Chromosomen-Menge fertig werden. Wie die praktische Erfahrung gezeigt hat, neigen Hybridzellen dazu, Chromosomen zu "verlieren". Bei jeder Zellteilung besteht bei der unphysiologischen Übermenge von Chromosomen die Gefahr, daß diese nicht gleichmäßig auf die beiden Tochterzellen verteilt werden. Die Tochterzelle, die weniger von dem Übermaß abbekommt und deshalb nicht mit Luxusproduktionen aufgehalten wird, hat gegenüber der anderen einen Selektionsvorteil und wird zur dominierenden Zelle in der Kultur. Die Synthese von Immunglobulin ist aber für die Lebensfähigkeit einer Hybridzelle nicht essentiell, sondern stellt eine "Luxus"-Synthese-Leistung dar. Das Erscheinen von Non-Producer-Varianten in einem Hybridoma-Klon ist deshalb ein häufiges Ereignis und erfordert aufwenige Re-Klonierungsmaßnahmen, um die Produktionsfähigkeit eines Klons zu sichern. Die Tendenz, Chromosomen zu verlieren, ist besonders extrem bei Inter-Spezies-Hybriden vorhanden.

3. Hybride Immunglobuline

Myelom-Zellen sind maligne B-Zellen und bilden selbst Immunglobuline (mit unbekannter Antigen-Bindungsspezifität). Diese Fähigkeit bringt die Myelomzelle wie die normale B-Zelle in das Hybridoma mit ein. Da die verschiedenen Ketten des Ig-Moleküls getrennt synthetisiert und erst nachträglich zu kompletten Antikörpern zusammengesetzt werden, entstehen in einer Hybridoma-Zelle, in der die zwei verschiedenen L- und H-Ketten synthetisiert werden, zufallsbedingt 10 verschiedene Kombinationen, von denen der gesuchte "richtige" Antikörper nur I/I6tel der Gesamt-Ig-Menge ausmacht. Deshalb sind mit großem Aufwand bereits Maus-Myelom-Zell-Mutanten entwickelt worden, die selbst keine H- oder L-Ketten bilden. Für die Fusion von menschlichen Lymphocyten steht aber bislang noch keine ähnlich weit entwickelte Myelom-Linie zur Verfügung.

Noch gravierendere Probleme liegen vor bei den Alternativen zur Hybridoma-Technik:

1. Immortalisierung von B-Lymphocyten durch Viren

Menschliche B-Lymphocyten von normalen Spendern können durch Infektion mit Epstein-Barr-Virus (EBV) maligne transformiert werden. Die EBV-infizierten, lymphoblastoiden Zellen können kontinuierlich in vitro kultiviert und kloniert werden. Im Vergleich mit Hybridoma produzieren EBV-lymphoblasoide Linien jedoch nur 1/10 oder weniger an Immunglobulinen bei unzufriedener Produktionsstabilität. Man nimmt an, daß B-Zellen in einem frühen Differenzierungsstadium durch EBV fixiert werden und deshalb überproportional häufig Klone erhalten werden, die IgM in sehr geringen Mengen produzieren.

In analoger Weise können Maus-B-Lymphocyten durch den Abelson-Mäuse-Leukämie-Virus (MuLV) transformiert werden. Auch hier werden die Lymphocyten ungünstigerweise in einem frühen Differenzierungsstadium fixiert und sind schlechte Antikörper-Produzenten. 2. Langzeitkulturen von nicht-transformierten B-Lymphocyten

Neueste Veröffentlichungen (Spredni, B. et al.: Long-term culture and cloning of nontransformed human B-lymphocytes. J. Exp. Med. 154, 1500-1516 (1981), Howard, M. et al.: Long-term culture of normal mouse B-lymphocytes. Proc. Natl. Acad. Sci. USA in press) zeigen die Möglichkeit auf, B-Lymphocyten ohne Transformierung durch spezielle Kulturbedingungen (permanente mitogene Stimulierung; Lymphokinkonditionierte Medien etc.) permanent zu kultivieren und zu klonieren. Für eine routinemäßige Herstellung von monoklonalen Antikörpern sind diese Verfahren derzeit mit Sicherheit noch nicht geeignet.

Der Stand der Technik kann daher zusammenfassend wie folgt beschrieben werden:

B-Lymphocyten von normalen Spendern können artifiziell "immortalisiert" werden. Die Hybridoma-Technik verwendet lebende, in Kultur unbegrenzt vermehrungsfähige Myelom-Zellen, die mit Antigen-stimulierten B-Lymphocyten fusioniert werden. Die durch Zell-Zell-Fusion geschaffenen Hybridzellen werden durch HAT-Selektion isoliert und durch Anlagen von Einzelzell-Kulturen kloniert. Hybridoma-Klone, die Antikörper mit der gesuchten Spezifität bilden, werden für die Massenproduktion von monoklonalem Antikörper vermehrt. Erhebliche Nachteile ergeben sich jedoch aus der HAT-Selektion, durch Chromosomen-Verluste und hybride Immunglobuline.

In einem anderen Verfahren werden B-Lymphocyten durch Infektion mit speziellen Viren maligne transformiert und in permanent wachsende Zellen umgewandelt, unter Erhalt der Antikörper-Synthese. Sie sind aber notorisch schwache Antikörper-Produzenten.

In Hinsicht auf Massenproduktion von monoklonalen Antikörpern mit definierter Antigen-Bindungsspezifität ist daher die Hybridoma-Technik den alternativen Verfahren derzeit eindeutig überlegen.

Aber auch die Hybridoma-Technik hat schwerwiegende Nachteile. Der wichtigste Nachteil besteht darin, daß das Verfahren einerseits auf HAT-sensitive Fusionspartner, andererseits auf wenige Zellarten, nämlich Lymphocyten und Nervenzellen beschränkt ist. Die Erfindung hat sich die Aufgabe gestellt, diese Nachteile zu eliminieren und ein neues, vorteilhaftes Verfahren zur Herstellung von permanent züchtbaren tierischen und humanen Zellinien zu schaffen.

Gelöst wird diese Aufgabe erfindungsgemäß durch ein Verfahren zur Gewinnung von permanent züchtbaren tierischen und humanen Zellinien durch Fusion von normalen tierischen und humanen Zellen mit biologischen Komponenten, welche eine Züchtungsfähigkeit in vitro bewirken, dadurch gekennzeichnet, daß man normale tierische und humane Zellen mit alleine nicht vermehrungsfähigen Zellfragmenten transformierter Zellen fusioniert und in einem Kulturmedium ohne Selektionssubstanzen züchtet.

Wesentlich beim Verfahren der Erfindung ist, daß die für die Fusion verwendeten Fragmente völlig frei sind von noch vermehrungsfähigen Zellen und sich alleine auch nicht mehr vermehren können. Überraschenderweise führt beim Verfahren der Erfindung das Übergewicht des Cytoplasmaanteils des nicht entarteten Partners, also der normalen Zelle, nicht zu einer Extinktion der malignen Eigenschaften der entarteten Zellen und damit zur Beseitigung der Fähigkeit zu permanentem Wachstum, obwohl es bekannt ist, daß durch Fusion von transformierten Zellen mit normalem Cytoplasma aus nichtmalignen Zellen die Malignität gelöscht wird (Shay, W.J. et al.: Supression of tumorigenicity in Cybrids. J. Supramol. St. Cell. Biochem. 16, 75-82 (1981)). Ebenso war nicht vorhersehbar, ob sich die Zellkerne mit den isolierten Kernen der entarteten Zellen, z. B. mit Myelomkernen, zu einem gemeinsamen Genom vereinigen, wenn das Myelom- Cytoplasma nicht mit in das Hybrid eingebracht wird.

Die Fusion erfolgt nach bekannten Methoden zweckmäßig in Gegenwart von fusiogenen Substanzen, vorzugsweise von Polyäthylenglycol oder von Sendai-Virus, da hierdurch analog wie bei der Hybridoma-Technik eine Erhöhung der Fusionsausbeute bewirkt wird. Weitere fusiogene Substanzen sind dem Fachmann bekannt und ebenfalls verwendbar.

Die Gewinnung der Fragmente der transformierten Zellen, beispielsweise also der Myelomzellen, kann nach bekannten Methoden erfolgen. Bevorzugt wird die Zellwand durch Lyse oder mechanisch aufgebrochen. Gegebenenfalls können dann durch Zentrifugieren die Kernfraktionen von den Cytoplasmafraktionen getrennt und die Fraktionen alleine verwendet werden. Besonders bevorzugt erfolgt die Lyse durch quellen lassen der Zellen in Glycerin und anschließendes Einbringen in glycerinfreie Pufferlösung. Dies führt zum Platzen der Zellmembranen. Eine andere bevorzugte Methode besteht in der Herstellung von Karyoplasten und Cytoplasten durch Behandlung der Zellen mit Cytochalasin B, einem im Handel erhältlichen Antibiotikum. Dieses Verfahren ist bekannt aus Biochem. Biophys. Res. Comm. 63, 669-674 (1975). Bei diesem Verfahren tritt eine Art "Zellteilung" auf, in den nur noch von der Zellmembran umgebenen Zellkern, den Karyoplasten einerseits und das ebenfalls von einer Membran umschlossene, kernlose Cytoplasma, den sogenannten Cytoplasten, andererseits. Beide erwiesen sich im Rahmen der Erfindung als in gleicher Weise für die Fusion geeignet wie durch Lyse oder mechanisch erhaltene Zellfragmente bzw. Kerne oder Cytoplasmafraktionen, die nicht mehr von einer Zellmembran umgeben sind. Der mechanische Aufschluß kann nach den dem Fachmann wohlbekannten Methoden, die hier keiner Erläuterung bedürfen, erfolgen.

Die Zellfragmente können in frischem Zustand unmittelbar nach ihrer Herstellung oder erst später für die Fusionierung eingesetzt werden, wobei sich in letzterem Fall eine Aufbewahrung in lyophilisiertem Zustand bewährt hat.

Unter transformierten Zellen werden solche verstanden, die in vitro und in vivo den normalen Wachstumsregelmechanismen nicht mehr gehorchen. Beispiele hierfür sind maligne entartete Zellen, wie z. B. Krebszellen, durch Virusinfektion (z. B. Eppstein-Barr-Virus) entartete Zellen und durch kanzerogene Stoffe veränderte Zellen.

Falls für das Verfahren der Erfindung Zellfraktionen verwendet werden, so müssen diese nicht völlig rein sein, sie dürfen jedoch keine intakten vermehrungsfähigen Zellen enthalten.

Ein wesentliches Merkmal der Erfindung liegt darin, daß die erhaltenen Hybride nicht der Konkurrenz von entarteten, nicht hybriden, in vitro züchtungsfähigen Zellen ausgesetzt sind und daher deren Wachstum auch nicht durch HAT-Selektionssubstanzen unterdrückt werden muß. Dadurch wird der sehr nachteilige Einfluß des HAT-Selektionsmediums auf die Hybride beseitigt und eine entscheidende Verbesserung der Ausbeute und Lebensfähigkeit an permanent züchtbaren Zellen erzeugt.

Ferner ist es erfindungsgemäß für die Hybridbildung nicht mehr Voraussetzung, als entartete Zelle eine HAT-sensitive Zelle zu verwenden. Es gibt viele permanent wachsende Zellinien, die keine HAT-Sensibilität aufweisen und für die konventionelle Hybridoma-Technik nicht verwendbar waren, im Rahmen der Erfindung aber zur Herstellung der Fragmente für die Fusion eingesetzt werden können.

Zellen werden durch Cytochalasin B (Pilz-Metabolit) veranlaßt, ihren Kern in einer extremen Ausstülpung zu "extrudieren". Unter Einfluß von Schwerkräften (z. B. Zentrifugation) reißt die dünne Verbindung leicht ab. Dadurch entsteht ein kernloser Zell-Leib (Cytoplast) und ein Kern, der mit Zellmembran und einem schmalen Cytoplasma-Saum umgeben ist (Karyoplast oder Minicell). Weder Karyoplast noch Cytoplast sind vermehrungsfähig, halten aber ihre speziellen Funktionen über einige Stunden bis Tage aufrecht. Die Kern-Extrusion erfordert eine relativ hohe Cytochalasin-B-Konzentration und ist, solange die Verbindung nicht abreißt, voll reversibel. Geringere Cytochalasin-B-Konzentrationen unterdrücken die Zellteilung nach Mitose ohne Kern-Extrusion. Die Standard-Methode für die Cytoplast/Karyoplast-Herstellung für nicht-adhärente Zellen ist in Wigler, M.H. and Weinstein, I.B.: preparative method for obtaining enucleated mammalian cells Biochem. Biophys. Res. Comm. 63, 669-674 (1975) beschrieben.

Es stellte sich ferner heraus, daß nicht nur oben erwähnte B-Lymphozyten sondern auch alle anderen bisher untersuchten tierischen und menschlichen Zellen sich erfindungsgemäß "immortalisieren" lassen (s. Beispiele 6 bis 8). Es konnten so unterschiedliche Zelltypen wie T-Lymphozyten (Träger der zellvermittelten Immunität und Regulatorzellen des Immunsystems), Endothelzellen (Wandzellen aus menschlichen Nabelschnurvenen) und Melanomzellen (aus kryopräserviertem Tumormetastasenmaterial isoliert) durch die erfindungsgemäße Methode in permanentes Wachstum überführt (immortalisiert) werden.

Damit macht es das erfindungsgemäße Verfahren möglich, beliebige tierische und menschliche Zellen in Kultur zu nehmen und auf diese Weise auch das Problem zu lösen, Zellprodukte wie z. B. Antikörper, Gerinnungsfaktoren, Enzyme und sonstige, von der Zelle synthetisierte Substanzen in vitro herzustellen. Ebenso machen es die erfindungsgemäßen Kulturen möglich, in erheblichem Umfange Versuchstiere für die Prüfung chemischer Substanzen entbehrlich zu machen.

Ein weiterer Gegenstand der Erfindung ist auch die Verwendung einer nach dem erfindungsgemäßen Verfahren hergestellten, permanent züchtbaren Zellinie zur Gewinnung von Zellprodukten wie monoklonalen Antikörpern, Gerinnungsfaktoren, Lymphokinen, Enzymen und sonstigen zu den Proteinen oder anderen Stoffgruppen gehörenden Zellprodukten.

Insbesondere kann man diese Ausführungsform der Erfindung bei Verwendung permanent züchtbarer B-Lymphozyten zur Herstellung monoklonaler Antikörper, bei Verwendung permanent züchtbarer Endothel-Zellen, Melanomzellen, Hepatozyten, Nierenzellen und dergleichen zur Gewinnung von Gerinnungsfaktoren, bei Verwendung permanent züchtbarer T-Lymphocyten + B-Lymphocyten oder/und Makrophagen zur Gewinnung von Lymphokinen, bei permanent züchtbaren Drüsenzellen zur Gewinnung der von den Drüsen sezernierten Produkte wie Hormone und dergleichen verwenden. Man erkennt, daß je nach der Art der für die Immortalisierung gemäß der Erfindung verwendeten tierischen Zellen alle interessierenden Zellprodukte gewonnen werden können, so daß es nicht erforderlich erscheint, diese hier detailliert aufzuführen.

Die Gewinnung von Zellprodukten ist jedoch nicht auf die Produkte der Ausgangszellen, also homologe Zellprodukte, beschränkt. Die erfindungsgemäß erhaltenen, permanent züchtbaren Zellinien können auch zur Expression von Zellprodukten verwendet werden, die von den Ausgangszellen nicht gebildet werden, also heterolog sind und deren genetische Information erst nach den Methoden der Genneukombination in die bereits permanente Hybridzelle eingebracht wird, die also heterolog sind. Die Herstellung heterologer Zellprodukte durch die permanente Hybridzelle kann z. B. durch Transformation bewirkt werden. So haben Versuche gezeigt, daß in die permanente Zelle gemäß der Erfindung mit Hilfe eines Vektors DNA eingeführt werden kann und somit zur Expression der durch die eingeführte DNA codierten Produkte verwendet werden kann.

Die erfindungsgemäßen permanent züchtbaren Zellen können darüberhinaus wie erwähnt auch als Prüfobjekte für Wirksubstanzen verwendet werden. Ferner können die erfindungsgemäß erhaltenen immortalisierten Hybridzellen auch als Quelle für die genetische Information, welche die Expression gewünschter Zellprodukte codiert, verwendet werden, derart, daß man den die genetische Information tragenden Bestandteil der Hybridzelle, also ihr Genom, Teile des Genoms oder RNA gewinnt und nach den Methoden der Genneukombination in einen geeigneten Mikroorganismus transformiert und das gewünschte Zellprodukt aus letzterem gewinnt.

Gemäß einer Abwandlung der erfindungsgemäßen Verwendung kann daher die Herstellung der Zellprodukte wie der monoklonalen Antikörper und anderer zellulärer Substanzen, auch so erfolgen, daß die gebildeten Hybridzellen nicht direkt für die Zellproduktbildung bzw. Substanzsynthese gezüchtet, sondern ihr Genom oder Teile des Genoms oder RNA nach den Methoden der Genneukombination in einen geeigneten Mikroorganismus transformiert und letzterer zur Gewinnung des monoklonalen Antikörpers oder der zellulären Substanzen gezüchtet wird. Bei dieser Ausführungsform der Erfindung isoliert man das Genom der Hybridzellen nach den dem Fachmann hierfür bekannten Methoden und transformiert es mit Hilfe eines geeigneten Vektors, wozu die handelsüblichen Vektoren verwendet werden können, nach den hierfür entwickelten Standardmethoden in einen geeigneten Mikroorganismus.

Der transformierte Mikroorganismus wird dann in üblicher Weise gezüchtet und das gewünschte Zellprodukt aus ihm gewonnen. Als Mikroorganismus wird vorzugsweise einer der für die Genneukombination bewährten E. coli-Stämme verwendet.

Die folgenden Beispiele erläutern die Erfindung weiter. Dabei werden folgende Abkürzungen und Warenbezeichnungen verwendet:

AK Antikörper

Ig Immunglobulin

H- bzw. L-Kette "Schwer"- bzw. "Leicht"-Proteinkette von Ig-Molekülen

Pristan 2, 6, 10, 14-Tetramethyl-pentadecan

HAT-Selektionsmedium Kulturmedium enthaltend Hypoxanthin, Aminopterin, Thymidin
TK Thymidin-Kinase
HGPRT Hypoxanthin-Guanin-Phosphoribosyl-Transferase
EBV Epstein-Barr-Virus
MuLV Abelson-Mäuse-Leukämie-Virus
CB Cytochalasin B Antibioticum (ALDRICH BIOCHEMICALS Milwaukee USA)
DMSO Dimethylsulfoxid
DMEM Dulbecco's Minimal Essential Medium
FKS Fetales Kälberserum
Ficoll polymerer Rohrzucker (PHARMACIA)
PEG Polyäthylenglycol
PBS phosphate buffered saline
POD Peroxidase
ABTS Ammoniumsalz von 2,2'-azino-di(3-ethylbenzothiazoline-6-sulfonic acid)
EBSS Earle's Balanced Salt Solution
RPMI 1640 Rosewell Park Memory Institut (Medium)
Tris Tris(hydroximethyl)aminomethan
PBL periphere Blut-Lymphozyten
MNC Mononucleäre Zellen (Lymphocyten, Monocyten)
hTSH Humanes Thyreoidea-stimulierendes Hormon
ß-hTSH β-Kette des...
FA Freund'sches Adjuvans
CFA komplettes Freund'sches Adjuvans
IFA inkomplettes Freund'sches Adjuvans
Methocel 1500 Methylcellulose (FLUKA)
CMV Cytoplasma-Membran-Vesikel
FITC-Covaspheres Fluoreszeinisothiocyanat in Kugelform (COVALENT TECHNICALS Ann Arbor, Mich. USA)
EAZ Ehrlich-Aszites-Zellen (ATCC; CCL 77)

### Beispiel 1

A Herstellung von Karyoplasten und Cytoplasten aus Maus-Myelomzellen der Linie P3X63 Ag 8.653 ATCC No-CRL-1580 (analog der in Wigler, M.H. and Weinstein, I.B.: A preparative method for obtaining enucleated mammalian cells. Biochem. Biophys. Res. Comm. 63, 669-674 (1975) beschriebenen Methode).

#### A.1 Material

Cytochalasin B (CB, Aldrich Biochemicals, Milwaukee, USA) wurde in Dimethylsulfoxid (DMSO, Merck) gelöst (2 mg/ml) und als Stammlösung bei 4 °C gelagert.

Ficoll-400 (Pharmacia; polymerer Rohrzucker) wurde in redestilliertem Wasser gelöst (1 g/ml), autoklaviert und als 50 %ige Stammlösung bei -20 °C gelagert.

Einfach- und doppelkonzentriertes Dulbecco's Minimum Essential Medium (DMEM), Fetales Kälberserum (FKS), L-Glutamin (200 mmol/l), Streptomycin-Penicillin von Boehringer Mannheim. Cellulose-Nitratröhrchen wurden durch UV-Bestrahlung sterilisiert. Myelomzell-Linie Ag 8.653 ATCC CRL-1580: Die Linie ist in Kearney, J.F. et al.: A new mouse myeloma cell line that has lost immunoglobulin expression but permits the construction of antibodysecreting hybrid cell lines. J. Immunol. 123, 1548-1550 (1979) beschrieben. Sie ist Azaguinin- resistent, HAT-sensitiv und synthetisiert weder H- noch L-Ig-Ketten. Sie wird in DMEM + 15 % FKS + Glutamin + Penicillin-Streptomycin + Pyruvat (= DMEM-Vollmedium) bei 37 °C in 7 % $CO_2$-Atmosphäre gehalten.

#### A.2 Methoden

Enukleierung: $8 \times 10^7$ Ag 8.653-Zellen wurden 5 min bei $10^3$ UpM zentrifugiert und in 12 ml einer 12,5 %igen Ficoll-DMEM-CB-DMSO-Lösung so lange resuspendiert, bis eine Zellklumpen-freie Suspension hergestellt war. Je 3 ml der Zellsuspension wurden auf 4, 12 Stunden vorher präparierte Ficoll-Gradienten, geschichtet und mit 2 ml Ficoll-freier DMEM-CB-DMSO-Lösung überschichtet. Die Röhrchen mit den Gradienten wurden in einer Ultrazentrifuge 60 Minuten lang bei 25.000 UpM (31 °C) zentrifugiert.

Nach Beendigung der Zentrifugation wurden die makroskopischen sichtbaren Fraktionen ("Banden") mit Hilfe einer Injektionsspritze mit langer Kanüle von oben her getrennt gesammelt, in je 20 ml Kulturmedium (DMEM ohne Zusätze) verdünnt, durch Zentrifugation sedimentiert und in frischem DMEM resuspendiert.

Folgende 4 Fraktionen wurden erhalten:
a) Zell-Debris (an der Grenze zwischen 0 und 12,5% Ficoll),
b) kernlose Cytoplasten im Bereich von 15 bis 16 % Ficoll),
c) Kerne ohne erkennbaren Plasmasaum und ca. 2 % kernlose "Zellen" aCn der Grenze zwischen 17 und 25 % Ficoll,
d) kernhaltige, nach morphologischen Kriterien intakte Zellen mit gut erkennbarem Plasmasaum und wenige Kerne ohne Plasmasaum als Sediment auf dem Röhrchenboden.

Die Zellzählung ergab, daß von den $8 \times 10^7$ Ag 8.653-Zellen in b) $1,25 \times 10^6$ Cytoplasten, in c) $4 \times 10^6$ Karyoplasten und in d) $1,1 \times 10^7$ mutmaßlich intakte Zellen enthalten waren.

B) Fusion von Maus-Milz-Zellen mit isolierten Myelom-Karyoplasten, -Cytoplasten und -Sedimentzellen aus Versuch A.

#### B.1 Material

Fusionsmittel: 20 g Polyethylenglycol (PEG-4000) wurden im Autoklaven geschmolzen, auf 56 °C abgekühlt und bei dieser Temperatur mit 20 ml DMEM vermischt.

HAT-Selektionsmedium: Zu DMEM-Vollmedium wurden Aminopterin ($4 \times 10^{-7}$ M), Thymidin ($1 \times 10^{-4}$ M) und Hypoxanthin ($3,1 \times 10^{-5}$ M) gegeben.

Kulturgefäße: Tissue Culture Cluster 24 und Cluster 96 der Firma Costar, Cambridge, Mass., USA.

#### B.2 Methoden

Fusionen: Die im Versuch A präparierten Fraktionen b), c) und d) wurden in getrennten Ansätzen mit Milzzellen im Verhältnis 10:1 vermischt und durch

Zentrifugieren sedimentiert. Die überstehende Flüssigkeit wurde sorgfältig entfernt. Zu dem Sediment wurden 0,8 ml 50 %ige PEG-Lösung (bei 37 °C, gleichmäßig über 1 Minute verteilt, unter ständigem, sanften Schütteln) und dann 5 ml DMEM (bei Raumtemperatur, gleichmäßig über 5 Minuten) gegeben. Nach Zugabe von weiteren 20 ml DMEM wurden die Zellen sedimentiert, in frischem DMEM-Vollmedium (5 ml) resuspendiert, und auf je 10, mit "feedercells" beschichte 24er Costar-Tüpfel Gewebskulturgefäße verteilt. Die Einzelkulturen wurden am Tag 1, 2, 3, 5, 7, 10, 13 mit DMEM-Vollmedium "gefüttert".

Feeder-cells ((Bauchhöhlen-Makrophagen): Am Tag vor der Fusion wurden Inzuchtmäuse (Balb/c) durch Streckung getötet. Unter sterilen Bedingungen wurden 4 bis 5 ml PBS in die Bauchhöhle gespritzt und nach 1 Minute wieder abgesaugt. Die ausgespülten Zellen wurden in DMEM gewaschen, in Vollmedium auf eine Dichte von $2 \times 10^5$ Zellen pro ml suspendiert und in 0,5 ml-Portionen auf 24er Costar-Tüpfel verteilt.

Milzzellen: Einer Balb/c-Maus wurde unmittelbar vor Fusion unter aseptischen Bedingungen die Milz exstirpiert und deren Zellen in DMEM suspendiert. Zellaggregate und Gewebsstücke wurden mit Mullgaze abfiltriert.

Elisa auf Maus-Immunoglobulin: Mikrotiterplatten wurden mit Maus-Ig-Antikörpern vom Schaf (IgG-Fraktion; 10 µg/ml 0,9 %ige NaCl- Lösung; 150 µl Antikörper-Lösung pro Tüpfel) beschichtet. Je 100 µl Kulturüberstand wurden in die beschichteten Tüpfel pipettiert und eine Stunde bei Raumtemperatur inkubiert. Nach Absaugen der Überstände und zweimaligem Waschen wurden die Tüpfel mit 100 µl anti-Maus-Ig-POD-Konjugat-Lösung (gleicher Antikörper wie oben; kovalent verbunden mit Meerrettich-Peroxidase) beschickt und eine Stunde bei Raumtemperatur inkubiert. Nach dreimaligem Waschen wurden pro Tüpfel 100 µl Substrat- Lösung (ABTS) pipettiert und die Farbentwicklung photometrisch bestimmt.

### B.3 Ergebnisse

Die gemäß A präparierten Cytoplast-, Karyoplast- und Sediment-Fraktionen wurden parallel mit Milzzellen einer Balb/c-Maus fusioniert und auf je 10 1-ml-Kulturen verteilt. Je 5 der Teilkulturen wurden ohne (Platte I) und je 5 mit HAT-Zusatz (Platte II) gefüttert.

Bis zum Tag 21 nach Fusion (n.F.) war in keinem der Tüpfel Wachstum von lymphoiden Zellen makroskopisch oder mikroskopisch nachweisbar, mit Ausnahme der Tüpfel 4A, 4B, 4C, 3C und 3D auf Platte I, die das Fusionat der Sedimentfraktion ohne HAT-Medium enthielten. In diesen Tüpfeln waren bereits 5 Tage nach Fusion (n.F.) Kolonien erkennbar, die sich rapide vergrößerten. Am Tag 8 n.F. wurde in diese Tüpfel HAT-Medium gegeben: innerhalb von 4 Tagen waren daraufhin alle sichtbaren Kolonien abgestorben.

Ab Tag 27 n.F. wurden zunächst vereinzelt, dann in nahezu allen Tüpfeln Kolonien sichtbar, die aus großen, kugeligen, transparenten, nichtadhärentwachsenden Zellen bestanden. Am Tag 65 n.F. waren mit Ausnahme von I-3B, II-1A, II-4A alle Tüpfel mit multiplen Kolonien von lymphoiden Zellen besetzt. Die Prüfung der Kulturüberstände auf Gehalt an Maus-Ig an diesem Tag ergab, wie die Zusammenstellung in Tabelle 1 zeigte, positive bis stark positive Werte im ELISA in allen Teilkulturen mit Ausnahme der obengenannten Kolonie-freien Tüpfel:

### Tabelle 1

ELISA zum Nachweis von Maus-Immunglobulin in den Kulturüberständen des Versuchs B (Tag 65 nach Fusion).

Tüpfel-Kulturplatte I: ohne HAT
(Ausnahmen: 4A, 4B, 4C, 3C, 3D: + HAT, Tag 8 bis 15)

|   | 1 | 2 | 3 | 4 | 5 | 6 |
|---|---|---|---|---|---|---|
| A | 664 | 601 | 706 | 632 |   |   |
| B | 526 | 766 | 011 | 633 |   |   |
| C | 576 | 769 | 855 | 623 |   |   |
| D | 794 | 1500 | 791 |   |   |   |

↑ 1  ↑ 2  ↑ 3

1 = Cytoplasten-Fusionat
2 = Karyoplasten-Fusionat
3 = Sedimentzellen-Fusionat

Tüpfel-Kulturplatte II:
mit HAT (Tag 1 bis 14)

|   | 1 | 2 | 3 | 4 | 5 | 6 |
|---|---|---|---|---|---|---|
| A | 008 | 568 | 580 | 000 |   |   |
| B | 267 | 538 | 539 | 547 |   |   |
| C | 656 | 530 | 729 | 822 |   |   |
| D | 848 | 570 | 605 |   |   |   |

↑ 1    ↑ 2    ↑ 3

negativ-Ktr. 1 (DMEM-Vollmedium):˙010
negativ-Ktr. 2 (DMEM ohne FKS):˙040
positiv-Ktr. 1 (Mausserum 1x10-3):˙723
positiv-Ktr. 2 (Mausserum 1x10 -2): >1500

a) Die Fusion von Karyoplasten mit Maus-Milz-Zellen führte zu in vitro vermehrbaren, Immunglobulin-sezernierenden Zellen. Obwohl nur ein kleiner Teil des Cytoplasmas des malignen Partners in das Hybrid eingebracht wurde, kam es nicht zu der möglichen Extinktion des Merkmals "permanentes Wachstum".

b) Die mit Karyoplasten erzeugten Hybridzellen synthetisierten und sezernierten Maus-Immunglobulin in "Hybridoma"-Quantitäten. Das Fehlen des Cytoplasma-Anteils der Ag 8.653 beeinträchtigte demnach die Produktions- und Sekretionsfähigkeit der Karyoplast-Milzzell-Hybride nicht.

c) Aus der Fusion von Cytoplasten mit Milzzellen gingen ebenfalls Zellklone hervor, die in vitro proliferierten und Antikörper sezernierten. Eine befriedigende Erklärung ist für dieses besonders überraschende Phänomen z. Z. nicht zu geben.

**Beispiel 2**

Zellfragmentierung mittels Glycerin-Lyse und Fusion mit humanen Blut-Lymphocyten

**Material**

Earle's Balanced Salt Solution (EBSS), Kulturmedium APMI 1640, Fetales Kälberserum (FKS) von Boehringer Mannheim. 8-Azaguanin (8-Ag) von Serva (Heidelberg), Agar (Bacto-Agar 1614) von Difco (Fa. Hedinger KG, Stuttgart). Die humane Plasmacytom-Linie HS SULTAN ATCC CRL-1484 wurde als kryopräserviertes Zellmaterial nach ATCC-Vorschrift aufgetaut und in Kultur genommen.

Nach dem in Proc. Natl. Acad. Sci. USA. 71, 2679-2683 (1974) beschriebenen Verfahren wurden $8 \times 10^7$ HS Sultan-Zellen in 100 ml RPMI 1640-Vollmedium, das 20 µM 8-Ag enthielt, 48 Stunden lang kultiviert. Die überlebenden Zellen wurden in 10 ml RPMI 1640-Vollmedium ohne 8-Ag aufgenommen und über 10 Tage vermehrt. Die Zellen wurden sodann auf Soft-Agar-Platten (COFFINO, P. et al: Proc. Natl. Acad. Sci. USA. 68, 219-223 (1971)), die mit RPMI 1640-Vollmedium + 20 µM 8-Ag hergestellt worden waren, ausgesät (ca. 500 Zellen pro Petrischale) und im $CO_2$-Inkubator bebrütet. Nach 9 Tagen wurden isoliert wachsende Kolonien steril von der Agaroberfläche entnommen und in RPMI 1640-Vollmedium + 8-Ag vermehrt. Ein Klon (als HS-SULTAN-8Ag-RI bezeichnet), der mit einer Verdopplungszeit von ca. 20 Stunden wuchs, wurde für den folgenden Versuch verwendet. Die HS-R1-Zellen waren HAT-sensitiv: Zellen, die in einer Dichte von 1 bis $5 \times 10^5$ pro ml HAT-Medium (RPMI 1640-Vollmedium mit 0,1 mM Hypoxanthin, 400 nM Aminopterin, 31 µM Thymidin) kultiviert wurden, vermehrten sich nicht und starben innerhalb von 7 Tagen vollständig ab.

Humane Lymphocyten (aus dem peripheren Blut: PBL): 300 ml Venenblut wurden steril in Heparin-Lösung (2 U/ml Blut) gesammelt und die Fraktion der mononukleären Zellen (MNC: Lymphocyten, Monocyten) mit Standard-Methoden isoliert. $3 \times 10^8$ MNC wurden in 100 ml RPMI 1640 x 10 % FKS suspendiert und zur Abtrennung der Monocyten 24 h in Kulturgefäßen bei 37 °C in 5 % $CO_2$-Atmosphäre inkubiert.

**Methoden**

Fragmentierung von HS-RI: Die Zellen wurden nach (Jett, M. et al.: Isolation and characterization of plasma membranes and intact nuclei from lymphoid cells. J. Biol. Chem. 252, 2134-2142 (1977)) mit Glycerin beladen und durch Inkubation in 10 mM Tris-HCl-Puffer lysiert. Die Kerne wurden durch Zentrifugation mit 200 g (10 Minuten, 4 °C) von den Membranvesikeln abgetrennt, die ihrerseits durch Zentrifugation bei 5000 g (40 Minuten, 4 °C) sedimentiert wurden.

**Fusion**

Ca. 1 x $10^8$ 729 HS-R1-Kerne wurden mit Human-Lymphocyten in RPMI 1640 im Verhältnis 1:1 suspendiert und, wie in Beispiel 1, B.2 beschrieben, mittels PEG fusioniert.

Das Sediment von Membranvesikeln aus etwa 1 x $10^8$ HS-R1-Zellen wurde mit einer Suspension von 1 x $10^7$ Human- Lymphocyten überschichtet und mittels PEG fusioniert.

In einem Kontrollansatz wurden ca. 2 x $10^7$ HS-R1-Kerne in RPMI 1640-Vollmedium (ohne HAT) aufgenommen und in 4 24er Costar-Tüpfeln im $CO_2$-Inkubator in Kultur genommen.

Alle Fusionate und die Kontroll-Kultur wurden auf Maus-Bauchhöhlen-Makrophagen wie in Beispiel 1 beschrieben, als feeder- cells kultiviert.

Nachweis von Human-Ig in Kulturüberständen: Mikrotiter-ELISA wie in Beispiel 1, B.2 beschrieben. Zur Beschichtung wurde immunadsorptiv gereinigtes antihuman-Ig vom Schaf verwandt. Die gleiche AK-Präparation wurde zur Herstellung des anti-human-Ig-POD-Konjugats verwandt. Der Schaf-AK reagierte mit allen menschlichen Ig-Klassen und zeigte keine Kreuzreaktivität mit bovinem oder murinem Ig.

**Ergebnisse**

Fragmentierung durch Glycerin-Tris-HCl-Lyse: Die Behandlung zerlegte HS-R1-Zellen in eine kernhaltige Fraktion, die bei 200 g sedimentierte und in eine kernlose Cytoplasma-Membran-Vesikel-Fraktion, die bei 5000 g sedimentierbar war. Unter dem Mikroskop waren in keiner der beiden Fraktionen intakte HS-R1-Zellen erkennbar. Die Kerne waren mit mehr oder weniger, unregelmäßig begrenzten Cytoplasma-Fetzen umgeben. Die Auszählung ergab eine Kern-Ausbeute von 85 %. Die Vesikel-Fraktion enthielt neben wenigem Debris massenhaft 0,5 bis 2 µm große Vesikel ohne erkennbare Kernbestandteile.

Die Kultur von 2 x $10^7$ Kernen in RPMI-Vollmedium (ohne HAT) führte in einem Beobachtungszeitraum von 12 Wochen zu keinem Wachstum von HS-R1-Zellen.

Kultur der Fusionate: Die Kern-Lymphocyten- und Cytoplasma-Vesikel-Lymphocyten-Fusionate wurden auf 96 bzw. 10 24er Costar-Tüpfel verteilt und je zur Hälfte mit und ohne HAT-Zusatz in RPMI-Vollmedium auf Maus-Makrophagen kultiviert. Ab der zweiten Woche nach Fusion wurden Kolonien von -lymphoiden Zellen sichtbar, die kontinuierlich an Größe zunahmen.

Produktion von humanem Immunglobulin: Am Tag 21 nach Fusion wurden Kulturüberstände (am Tag 19 war ein kompletter Medium-Wechsel vorgenommen worden) auf Gehalt an menschlichem Immunglobulin geprüft. Die Ergebnisse zeigen die Tabellen 2a und 2b.

**Tabelle 2a**

ELISA zum Nachweis von Human- Immunglobulin in Kulturüberständen (Karyoplasten-Fusion, 21 Tage nach Fusion).

I + HAT

|   | 1 | 2 | 3 | 4 | 5 | 6 |
|---|---|---|---|---|---|---|
| A | 029 | 147 | 286 | 147 | 228 | 270 |
| B | 171 | 086 | 050 | 144 | 846 | 121 |
| C | 118 | 156 | 250 | 082 | 179 | 059 |
| D | 120 | 1148 | 024 | 096 | 023 | 151 |

I − HAT

|   | 1 | 2 | 3 | 4 | 5 | 6 |
|---|---|---|---|---|---|---|
| A | 660 | 233 | 175 | 270 | 410 | 322 |
| B | 103 | 264 | 229 | 253 | 271 | 260 |
| C | 045 | 343 | 370 | 128 | 150 | 126 |
| D | 171 | 173 | 141 | 116 | 218 | 699 |

II + HAT

|   | 1 | 2 | 3 | 4 | 5 | 6 |
|---|---|---|---|---|---|---|
| A | 135 | 290 | 107 | 279 | 530 | 674 |
| B | 116 | 500 | 469 | 315 | 315 | 627 |
| C | 120 | 050 | 068 | 159 | 226 | 145 |
| D | 191 | 078 | 686 | 110 | 242 | 561 |

II − HAT

|   | 1 | 2 | 3 | 4 | 5 | 6 |
|---|---|---|---|---|---|---|
| A | 381 | 235 | 489 | 514 | 608 | 217 |
| B | 348 | 239 | 214 | 158 | 367 | 163 |
| C | 185 | 275 | 235 | 234 | 322 | 316 |
| D | 219 | 202 | 292 | 283 | 220 | 052 |

**Tabelle 2b**

Cytoplasten-Fusion, 21 Tage nach Fusion.

|   | 1 | 2 | 3 | 4 | 5 | 6 |
|---|---|---|---|---|---|---|
| A | 052 | 627 | 917 |  |  |  |
| B | 041 | 326 | 715 |  |  |  |
| C | 071 | 400 | 826 | 0889 |  |  |
| D | 079 | 917 | 494 | 1016 |  |  |

    ↑       ↑          ↑
    1       2          3

1 = Kern-Kontroll-Kultur
2 = mit HAT
3 = ohne HAT

Setzt man die Extinktionswerte von 0 bis 99 als negativ bis zweifelhaft, die Werte von 100 bis 200 als positiv, die Werte > 200 als stark positiv an, dann ergibt sich für die durch Kernfusion erzeugten Kulturen folgende Verteilung:

Die Ergebnisse lassen folgendes erkennen: Fusionate, die mit Lyse-Fraktionen hergestellt werden, können ohne HAT-Selektion kultiviert werden. Das Verfahren ist sehr viel weniger arbeitsaufwendig als die Cytochalasin-B-Extrusion und liefert fusionsfähiges Material in hoher Ausbeute. Eine klare Auftrennung in "Kern-" und "Cytoplasma-Membran"-Fraktionen wird mit keinem der beiden Verfahren erreicht. Sowohl die überwiegend Kern-Material enthaltende wie die überwiegend Cytoplasma-Membran-Vesikel enthaltende Fraktion erzeugt nach Fusion mit menschlichen Lymphocyten aus dem Blut in vitro vermehrungsfähige, AKproduzierende Zell-Klone. Der Parallel-Ansatz der Kern-Lymphocyten-Hybride mit und ohne HAT-Zusatz dokumentiert die negativen Auswirkungen des Selektionsmediums: Mit HAT sind 23 % der Primärkulturen Ig-negativ und nur 40 % stark positiv; ohne HAT sind über 70 % stark positiv und nur 4 % Ig-negativ.

**Beispiel 3**

Fusion von Milzzellen einer immunisierten Maus mit nativen und fragmentierten Myelomzellen Ag 8.653

**Material**

Humanes Thyreoidea-stimulierendes Hormon (hTSH) und dessen isolierte β-Kette (ß-hTSH) stammten von Boehringer Mannheim. Komplettes und Inkomplettes Freund'sches Adjuvans (CFA, IFA) von Difco, Methocel 1500 von Fluka und FITC-Covaspheres von Covalent Tech. Co., Ann. Arbor, Michigan, USA.

| Kern-Lymphocyten-Hybride, kultiviert | ELISA auf Human-Immunglobulin | | |
|---|---|---|---|
|  | negativ | positiv | stark positiv |
| n | 11 | 18 | 19 |
| mit HAT (n = 48)   (%) | (23) | (37) | (40) |
| n | 2 | 12 | 34 |
| ohne HAT (n = 48)   (%) | (4) | (25) | (71) |

**Methoden**

Immunisierung: Balb/c-Mäuse wurden mit ßhTSH (40 µg in CFA, intraperitoneal) primär immunisiert (Tag 1), und am Tag 196 mit hTSH (50 µg in IFA, i.p.), am Tag 266 mit hTSH ohne Adjuvans i.p. sowie am Tag 294 mit hTSH intravenös "geboostert".

Milzzellen (ca. 1 x 10⁸) wurden von einer der immunisierten Mäuse 3 Tage nach der letzten Booster-Immunisierung, wie in Beispiel 1, B.2 beschrieben, gewonnen und je zur Hälfte für zwei Fusionen verwandt. Fusion 1: 5 x 10⁷ Milzzellen und 1 x 10⁷ Ag 8.653-Zellen wurden vermischt, wie in Beispiel 1, B.2 beschrieben, fusioniert und in 48 24er Tüpfel mit Maus-Makrophagen-Zellen in HAT-haltigem DMEM-Vollmedium kultiviert.

Fusion 2: 1 x 10⁷ Ag-8.653-Zellen wurden wie in Beispiel 2 beschrieben, durch schrittweise Behandlung mit Glycerin und 10 mM Tris-HCl-Puffer lysiert. Die Cytoplasma-Membran-Vesikel-(CMV-)Fraktion wurde pelletiert (5.000 g, 40 Minuten, 4 °C). Die Kern-Fraktion wurde mit 7 x 10⁷ Milzzellen vermischt, durch Zentrifugation auf das CMV-Sediment geschichtet und mittels PEG nach dem Standardverfahren, wie in Beispiel 1, B.2 beschrieben, fusioniert. Das Fusionat wurde im DMEM-Vollmedium auf 24 24er Costar-Tüpfeln mit Makrophagen erteilt und ohne HAT-Zusätze kultiviert.

Antigenspezifische Markierungen von Hybridzellen und Klonierung: (FITC-) Covaspheres wurden nach der allgemeinen Vorschrift des Herstellers mit hTSH kovalent beschichtet (TSH-CS) und in 5 o/oo Natriumazid-Lösung gelagert. Nach dem in PARKS, D.R. et al.: Proc. Natl. Acad. Sci. USA 76, 1982-1966 (1979) beschriebenen Verfahren wurden die Zellen mit den beschichteten Covaspheres markiert und mit Hilfe eines Cytofluorographen große, Fluoreszenz-positive Zellen einzeln in Tüpfel und 96er Costar-Platten "abgelegt". Die Tüpfel waren 24 Stunden zuvor mit Maus-Makrophagen in DMEM-Vollmedium beschicht worden.

ELISA für TSH-spezifische Antikörper: Beschichtung, Inkubation der Kulturüberstände, Substrat-Reaktion und Ablesung wie in Beispiel 1, B.2, beschrieben. Anstelle von anti-Maus-Ig-POD wurde TSH-POD-Konjugat verwendet. Als positive Kontrolle wurde Serum einer hTSHhyperimmunisierten Maus, 10⁻³ verdünnt, eingesetzt; als Negativ-Kontrolle diente ein Klon, welcher Antikörper gegen ein nichtverwandtes Antigen bildete (Maus-anti-Digoxin).

**Ergebnisse**

Sowohl in den Kulturen aus Fusion 1 (mit intakten Ag 8.653-Zellen) als auch denen aus Fusion 2 (mit Ag 8.653-Lyse-Fragmenten) waren am Tag 14 in allen Tüpfeln Kolonien von großen, lymphoiden Zellen erkennbar. Der mit Kulturüberständen vom Tag 14 durchgeführte ELISA zum Nachweis von TSH-spezifischen Antikörpern ergab die in Tabelle 3 zusammengefaßten Werte: In allen Teilkulturen wurde anti-TSH nachgewiesen.

**Tabelle 3**

a)

| | 1 | 2 | 3 | 4 | 5 | 6 |
|---|---|---|---|---|---|---|
| A | 235 | 236 | 212 | 149 | 119 | 212 |
| B | 109 | 221 | 119 | 104 | 176 | 068 |
| C | 116 | 108 | 135 | 139 | 093 | 135 |
| D | 171 | 128 | 120 | 228 | 137 | 145 |

b)

| | 1 | 2 | 3 | 4 | 5 | 6 |
|---|---|---|---|---|---|---|
| A | 271 | 268 | 267 | 286 | 194 | 291 |
| B | 288 | 278 | 362 | 317 | 280 | 305 |
| C | 207 | 292 | 252 | 226 | 292 | 271 |
| D | 295 | 376 | 370 | 338 | 310 | 333 |

Negativ-Kontrolle (DMEM-Vollmedium): 000
Positiv-Kontrolle (Anti-TSH-Mausserum): 362
ELISA zum Nachweis von Anti-TSH in Kulturüberständen am Tag 14 nach Fusion. a) Fusion 1 (intakte Ag8.653), b) Fusion 2 (Ag653-Fragmente)

Am Tag 15 wurden die nicht-adhärenten Zellen aus den Einzel-Tüpfeln von Fusion 1 und 2 herausgespült und in getrennten Ansätzen TSH-Antigen-spezifisch markiert (Grundlage: Hybridoma tragen in der Regel wie B-Lymphocyten einen Teil der von ihnen synthetisierten Antikörpermoleküle in der Zellmembran verankert, mit "nach außen" gerichteten Antigen-Bindungsstellen) und mit Hilfe des Zellsorters kloniert.

## Beispiel 4

Fusion von Humanen PBL mit intakten und mit fragmentierten Ag 8.653-Zellen.

## Material

Inaktiviertes Hepatitis-B-Surface-Antigen ($HB_{si}$; Biotest) wurde durch Immunadsorption von Serumproteinen gereinigt. ELISA zum Nachweis von humanen $HB_s$-Antikörpern: Mikrotiter-Platten wurden mit gereinigtem $HB_{si}$ (20 ug/ml 0,9%ige NaCl-Lösung) beschichtet. Inkubation der Kulturüberstände, Konjugat- und Substrat-Reaktion sowie Ablesung wie im Beispiel 1.B.2 beschrieben. Anstelle von Anti-Maus-Ig-POD wurde (Schaf-)Anti-Human-Ig-POD verwendet.

## Durchführung

HPBL von einem Spender mit hohem anti-HBs-Titer wurden aus 200 ml Venenblut durch Ficoll-Gradienten-Zentrifugation isoliert. Zur Anreicherung der B-Lymphocyten wurden die T-Zellen mit Schaferythrocyten (nach Standardverfahren) rosettiert und mittels einer zweiten Ficoll-Gradienten-Zentrifugation abgetrennt. Die Fraktion der nicht-rosettierenden Zellen (HPBL(B)) wurden in einer Dichte von $5 \times 10^7$ Zellen in RPMI 1640 + 10 % autologem Plasma (30 min/56 °C-hitzeinaktiviert) mit ca. 10 μg HBsi im $CO_2$-Inkubator kultiviert (Mediumwechsel alle 12 Stunden). Fusion 1: $1 \times 10^7$ der vorbehandelten HPBL(B) wurden mit $1 \times 10^7$ Ag 8.653 vermischt und, wie in Beispiel 1, B.2 beschrieben, mittels PEG fusioniert. Das Fusionat wurde in RPMI 1640 + 10 % Humanplasma + HAT in 4 24er Costar-Tüpfeln kultiviert. Fusion 2: $1,1 \times 10^8$ Ag 8.653-Zellen wurden mittels Glycerin-Lyse fragmentiert. $1 \times 10^7$ Ag 8.653-Kerne wurden mit $1 \times 10^7$ HPBL(B) vermischt und auf das Sediment von Membran-Cyptoplasma-Vesikeln (aus $1 \times 10^8$ Ag 8.653-Zellen) geschichtet. Nach Fusion mit PEG wurde das Fusionat in 4 24er Costar-Tüpfeln in RPMI 1640 + 10 % Humanplasma (ohne HAT-Zusatz) kultiviert.

## Ergebnisse

In allen Tüpfeln von Fusion 1 und 2 trat ab Tag 3 starkes Wachstum von sehr großen, adhärenten Zellen auf. Am Tag 5 wurde die Hauptmasse der nicht-adhärenten Zellen vorsichtig suspendiert und auf zwei neue 24er Costar-Tüpfel verteilt (z. B. A1 B1 und C1). Am Tag 28 in Fusion 1: kleine Kolonien in A2 und A3, in den restlichen Tüpfeln nur adhärente Zellen; in Fusion 2: massives Wachstum multipler Kolonien in allen Tüpfeln mit Ausnahme von C3. Der mit Kulturüberständen vom Tag 35 durchgeführte ELISA zum Nachweis von humanen Immunglobulinen mit Spezifität gegen Hepatitis-B-Antigen ergab das in Tabelle 4 zusammengefaßte Ergebnis: die Fusion 1 (intakte Ag 8.653-Zellen, Kultur in HAT-Medium) lieferte keine positive Kultur; dagegen waren 5 von 12 Kulturen der Fusion 2 (Ag 8.653-Fragmente, Kultur in Normal-Medium) eindeutig Anti-$HB_s$-positiv.

**Tabelle 4**

| a) | 1 | 2 | 3 | 4 | b) | 1 | 2 | 3 | 4 |
|---|---|---|---|---|---|---|---|---|---|
| A | 000 | 000 | 000 | 000 | A | 010 | 000 | 189 | 55 |
| B | 000 | 001 | 011 | 000 | B | 000 | 003 | 198 | 03 |
| C | 005 | 000 | 000 | 004 | C | 000 | 173 | 000 | 1C |

ELISA zum Nachweis von Human-Anti-$HB_s$ in Kulturüberständen am Tag 35 nach Fusion. a) Fusion[1] (intakte Ag 8.653), b) Fusion 2 (Ag 8.653-Fragmente).

Negativ-Kontrolle (Anti-$HB_s$-neg. Humanserum): 000

Positiv-Kontrolle (Anti-$HB_s$-pos. Humanserum): 185

## Beispiel 5

Fusion von HPBL mit Fragmenten aus der humanen Plasmacytom-Linie HS SULTAN

## Material

HS Sultan wurde von der American Type Culture Collection (ATCC) unter dem Code CRL-1484 als kyropräserviertes Zellmaterial bezogen und nach ATCC-Vorschrift aufgetaut und in Kultur genommen.

## Durchführung

HPBL wurden wie in Beispiel 4 beschrieben vom gleichen Spender aufgearbeitet und in vitro mit HBsi "geboostert".

Fusion: $4 \times 10^7$ HS SULTAN-Zellen wurden mittels Glycerin-Lyse fragmentiert. Die Membran-Cytoplasma-Vesikelfraktion wurde bei 5500 g (40 Minuten) sedimentiert und ein Gemisch von ca. $4 \times 10^7$ HS-SULTAN-Kernen und $4 \times 10^7$ HPBL(B) daraufgeschichtet. PEG-Fusion erfolgte nach Beispiel 4. Aussaat des Fusionats auf 12 24er Costar-Tüpfel in RPMI 1640 + 20 % FKS + Pyruvat + Insulin (Novo 2 U/ml) + 1 % nichtessentielle Aminosäuren (BM) + 1 % Methocel 1500 ohne HAT-Zusatz.

**Ergebnisse**

Tag 14 nach Fusion: Wachstum von großen, nichtadhärenten, lymphoiden Zellkolonien.

**Beispiel 6**

Immortalisierung von menschlichen T-Lymphozyten

6.1 Material und Methoden

T-Lymphozyten wurden mittels Standardverfahren (Rosettierung mit Schaferythrozyten; Ficoll-Gradientenzentrifugation) aus der Lymphozyten-Gesamtfraktion isoliert und sofort oder nach 3tägiger Kultur behandelt. Ehrlich-Aszites-Zellen (ATCC; CCL 77) wurden in DMEM mit 10 % Pferdeserum kultiviert und dienten als Spender der transformierenden Fragmente. Die Fragmentierung der EAZ wurde nach Jett et al. (J. Biol. Chem. 252, 2134-2142 (1977)) mittels Glycerin-Lyse ausgeführt. Die überwiegend Kernmaterial enthaltende Fraktion wurde durch Zentrifugation abgetrennt und verworfen. Die Mitochondrien-reiche cytoplasmatische Membran-Vesikelfraktion (CMV) wurde zur Transformierung verwandt. $5 \times 10^7$ T-Lymphozyten wurden mit einem Überschuß an PHA-Lektin (Difco) beladen, mit der CMV-Fraktion aus EAZ vermischt und 20 Minuten bei Raumtemperatur inkubiert. Die Mischung wurde durch Zentrifugation sedimentiert, der flüssige Überstand restlos entfernt und durch 1 ml einer 50%igen PEG-Lösung ersetzt. Nach 1minütiger Einwirkungszeit wurde die PEG-Lösung durch Zugabe von RPMI-1640-Kulturmedium ausverdünnt und durch Zentrifugation von den Zellen abgetrennt. Die Zellen wurden in RPMI-Medium mit 20 % foetalem Kälberserum (FKS, BM) aufgenommen, aus 12 1-ml-Kulturtüpfel verteilt und bei 37 °C in 5%iger CO$_2$-Atmosphäre kultiviert. Die Charakterisierung der Zellen anhand von Oberflächenmerkmalen erfolgte nach Standardverfahren mittels Schaferythrozyten (E)-Rosettierung (KAPLAN, M.E. et al.; J. Immunol. Methods 5, 131 (1974)) und mittels Immunfluoreszenz unter Verwendung der beiden T-Zell-spezifischen Antikörper OKT-3 (Ortho; REINHERZ, E.L. et al.; J. Immunol. 123, 1312 (1979)) bzw. MAK 4-11 (RIEBER, P. et al.; Hybridoma 1, 59 (1981)) und fluoreszenzmarkiertem Anti-Human-Immunglobulin (Ig; Fa. Dako) zum Nachweis von B-Zell-typischem Membran-Ig.

6.2 Ergebnisse

Innerhalb der ersten 14 Tage starb der Großteil der Zellen in den Kulturen ab. Ab Tag 21 wurden im Zelldebris Kolonien von kleinen bis mittelgroßen Zellen sichtbar, die sich kontinuierlich vermehrten. Wachstum von Kolonien wurden in allen 12 Tüpfeln gefunden, und zwar bis zu 50 Kolonien pro Tüpfel. Am Tag 30 wurden die Kolonien in größere Kulturgefäße überführt und weiter vermehrt. Die Zellen wurden anhand ihrer Oberflächen-Marker analysiert und folgende Ergebnisse erhalten:
a) E-Rosetten-positiv: > 95 %
b) OKT-3-positiv: > 90 %
c) 4-11-positiv: > 90 %
d) Ig$_s$-positiv: < 5 %
6.3 Beurteilung
Fragmente, die aus EAZ (permanente Mauszell-Linie) durch Glycerin-Lyse gewonnen werden können, erzeugen nach PEG-Fusion mit isolierten T-Lymphozyten permanent in Kultur wachsende Zellen, die aufgrund ihrer Oberflächenmerkmale eindeutig als T-Lymphozyten identifizierbar sind.

**Beispiel 7**

Immortalisierung von menschlichen Endothelzellen

7.1 Material und Methoden

Alle Kulturgefäße wurden vor Einsaat von Zellen mit Gelatine beschichtet. Das Kulturmedium bestand aus einer 1:1-Mischung von RPMI-1640 und Medium 199 (BM) mit 20 % FKS. Menschliche Endothelzellen wurden nach Jaffe et al. (J. Clin. Invest. 52, 2745-2756 (1973)) mittels Collagenase-Lösung (Gibco) aus den Venen von frischen Nabelschnüren gewonnen und vor der Transformierung durch Anlage einer Primärkultur über ca. 14 Tage vermehrt. Für die Transformierung wurden die adhärent wachsenden Endothelzellen mittels Trypsin-EDTA-Lösung (BM) abgelöst und in Suspension, wie unter 6.1 beschrieben, mit der CMV-Fraktion aus EAZ fusioniert. Die so behandelten Zellen wurden in einer Zelldichte von $5 \times 10^5$ pro 75 cm$^2$-Kulturgefäß ausgesät und im CO$_2$-Inkubator kultiviert. Zur Kontrolle wurden jeweils ein Aliquot der Endothelzellen aus den Primärkulturen ohne CMV-Fraktion mit PEG-Losung behandelt (Scheinfusion) und rekultiviert. Sobald die Zellen auf dem Boden des Kulturgefäßes einen lückenlosen Zellrasen ausgebildet hatten, wurden sie mittels Trypsin-EDTA-Lösung abgelöst und im Verhältnis 1:3 auf frische Kulturgefäße übertragen (= Passage).

7.2 Ergebnisse

Die mit CMV-Fraktion fusionierten Endothelzellen hafteten nach Einsaat mit einer Effizienz von 20 bis 30 % an und wuchsen innerhalb von zwei bis drei Tagen zu einem konfluenten Zellrasen aus. Die scheinfusionierten Zellen hafteten mit etwa der gleichen Effizienz an, vermehrten sich aber kaum und starben - ohne einen konfluenten Zellrasen auszubilden - innerhalb von ca. 21 Tagen vollständig ab. Gänzlich unbehandelte Endothelzellen vermehrten sich bis maximal in die 3. Passage, stellten dann das Wachstum ein, lösten sich unter Abkugelung vom Boden des Kulturgefäßes und lysierten. In 8 verschiedenen Ansätzen mit Endothelzellen aus insgesamt 18 verschiedenen Nabelschnüren wuchsen die CMV-behandelten Zellen problemlos über die 10. Passage hinaus. Die transformierten Endothelzellen konnten ohne Verlust an Lebens- und Teilungsfähigkeit in flüssigen Stickstoff ein- und ausgelagert werden.

7.3 Beurteilung

Humane Umbilical-Endothelzellen konnten ohne transformierende Manipulation gemäß Erfindung nicht über die 3. Passage kultiviert werden, womit die in der Literatur mitgeteilten Erfahrungen (z. B. Grimbrone, M.A., in Progress in Haemostasis and Thrombosis, Vol. 3; Maciag, T. et al., J. Cell Biol. 91, 420-426 (1981)) bestätigt wurden. Durch Fusion mit der Mitochondrienreichen CMV-Fraktion aus Ehrlich-Aszites-Zellen wurden die Kultureigenschaften der Endothelzellen so verändert, daß sie problemlos über die kritische 3. Passage hinaus vermehrbar waren (und sich derzeit in der 23. Passage befinden, ohne "Ermüdungserscheinungen" zu zeigen).

**Beispiel 8**

Immortalisierung von menschlichen Melanomzellen

8.1 Material und Methoden

Melanomzell-haltiges Gewebe wurde durch chirurgische Exzidierung einer Hüft- Lymphknoten-Metastase gewonnen, unter sterilen Bedingungen in kleine Würfel zerschnitten und bis zur Fusion in flüssigen Stickstoff eingelagert. CMV-Fraktionen aus EAZ wurden, wie unter 6.1 beschrieben, hergestellt.

Vor der Fusion wurde das Melanomzell-haltige Material aufgetaut und mittels Trypsin- Behandlung einer Einzelzell-Suspension hergestellt. Die Fraktion der großen, braun-rot pigmentierten Melanomzellen wurden durch Ficoll-Gradienten-Zentrifugation von den Begleit- Lymphozyten befreit und, wie unter 6.1 beschrieben, mit CMV-Fraktion unter PEG-Einfluß fusioniert (ca. $4 \times 10^5$ Melanomzellen mit CMV aus ca. $1 \times 10^6$ EAZ). Zur Fusionskontrolle dienten $4 \times 10^5$ Melanomzellen, die ohne CMV mit PEG behandelt wurden. Die Zellen wurden nach der Fusion in einer Dichte von $1 \times 10^5$ Zellen pro Tüpfel in RPMI 1640 + 20 % FKS ausgesät und bei 37° C in 5%iger $CO_2$-Atmosphäre kultiviert.

8.2 Ergebnisse

In allen 4 Teilkulturen der mit CMV fusionierten Melanomzellen wuchsen ab Tag 28 Kolonien von typisch pigmentierten Zellen in Form von semiadhärenten Zellhaufen, die sich kontinuierlich vergrößerten. In der Kontroll-Kultur der scheinfusionierten Zellen kam es zu keiner Zellvermehrung, vielmehr zeigten die Zellen ab Tag 8 eine zunehmende Granulierung, und am Tag 22 waren nur noch Zelltrümmer in der Kultur vorhanden.

8.3 Beurteilung

Humane Melanomzellen aus kryopräserviertem Metastasengewebe konnten durch CMV-Fusion zu in vitro Kultur- und vermehrungsfähigen Zellen transformiert werden. Scheinfusionierte Melanomzellen gleicher Herkunft starben dagegen bei sonst identischen Kulturbedingungen ab.

**Beispiel 9**

Einführung eines eukaryontischen DNA- Vektors in immortalisierte menschliche Endothel- Zellen

9.1 Nachweis von transfiziertem Material in den Zellen durch Abklatschen (Southern blotting) von Hirt-Überständen.

9.1.1 Material

Plasmid Z-pBR 322/RchrßG-Δ425 B (Dierks, P. et al., Proc. Natl. Acad. Sci. USA 78, 1411-1415 (1981)) enthält ein 2.070 Basenpaare (Bp) Kaninchen-β-globin-Genfragment.

Das Cla-Pvu I-Fragment von pBR 322 wurde durch ein 3.039 Bp Hpa I-Bam H 1-Fragment ersetzt, welches die gesamte Region der frühen Gene und den Anfang der Region der späten Gene von SV 40 enthält (Tooze, J. (1980), in "DNA Tumor Viruses", J. Tooze, ed., 2nd edition, Cold Spring Harbor Laboratory und B. Wieringa et el., Cetus-UCLA Symposium on Gene regulation 1982). Dieses Plasmid wird im folgenden als pBR 322 RßG SV 40 bezeichnet.

9.1.2 Methoden

Menschliche Endothelzellen wurden, wie im Beispiel 7 beschrieben, immortalisiert und wachsen gelassen. Je $10^6$ Zellen wurden mit 6 μg pBR 322 RßG SV 40 und mit 4 μg mit Ultraschall behandelter Kalbsthymus DNA transfiziert unter Anwendung der Kalziumphosphatfällungsmethode (Graham, F.L. et al., Virology 52, 456-467 (1973) und Wigler, M. et al., Cell 14, 725-731 (1978)). 10 Stunden und 40 Stunden nach der Transfektion wurden Hirt- Überstände hergestellt (Hirt, B., J. Mol. Biol. 26, 365-369 (1967)).

Die Hirt-Überstände wurden auf einem 1%igen Agarosegel getrennt und nach der Methode von Southern (Southern, E.M., J. Mol. Biol. 98, 503-517 (1975)) auf Nitrocellulosepapier übertragen. Plasmid pBR 322 RßG SV 40 wurde mit $^{32}$P unter Anwendung der Nick-Translationsmethode von Rigby, P.W. et al., J. Mol. Biol. 113, 237-251 (1977) markiert und mit der übertragenen DNA auf den Nitrocellulosefiltern hybridisiert wie beschrieben bei Maniatis, T. et al., Molecular Cloning, Cold Spring Harbor Laboratory (1982). Der Filter wurde auf Röntgenfilm bei -70° C belichtet.

9.1.3 Ergebnisse

Menschliche Endothelzellen, die mit Plasmid pBR 322 RßG SV 40 transfiziert wurden, ergeben ein starkes Hybridisierungssignal, welches dem authentischen Plasmid entspricht hinsichtlich seiner Mobilität auf Agarosegel. Ein Vergleich der Signale der Hirt-Überstände der Zellen 40 Stunden nach der Transfektion mit denen der Zellen 10 Stunden nach der Transfektion ergab eine vier- bis fünffache Steigerung der Intensität des Hybridisierungssignals.

Eine Hybridisierung an DNA-Spezies, die kleiner sind als das eingebrachte Plasmid wurde ebenfalls beobachtet. Diese Signale waren in nicht transfizierten immortalisierten menschlichen Endothelzellen nicht feststellbar.

Die gleiche Intensitätsverteilung der Hybridisierungssignale wurde beobachtet, wenn HeLa-Zellen mit Plasmid pBR 322 RßG SV 40 transfiziert wurden.

9.1.4 Beurteilung

Die Tatsache, daß in den Hirt-Überständen menschlicher immortalisierter Endsthelzellen die mit Plasmid pBR 322 RßG SV 40 transfiziert worden waren, eine DNA-Hybridisierung an $^{32}$P-markiertem eingebrachtem Plasmid festgestellt wurde, jedoch nicht bei nichttransfizierten Zellen, ist ein Beweis dafür, daß der Vektor in die eukaryontische Zellinie eingeführt wurde. Die Beobachtung, daß das Hybridisierungssignal bei transfizierten Zellen 40 Stunden nach der Transfektion vier- bis fünffach stärkere Intensität aufwies als bei Zellen 10 Stunden nach der Transfektion, läßt die Schlußfolgerung zu, daß das transfizierte Plasmid in den Zellen repliziert wurde.

9.2 Nachweis von Kaninchen-β-globin spezifischen Transcripten in immortalisierten menschlichen Endothelzellen, die mit Plasmid pBR 322 RßG SV 40 transfiziert wurden.

9.2.1 Material

Als Nuklease $S_1$, $T_4$ Polynukleotidkinase und Kälberdarmphosphatase wurden die handelsüblichen Präparate von Boehringer Mannheim verwendet.

9.2.2 Methoden

Immortalisierte menschliche Epithelzellen wurden wie in 9.1.2 beschrieben, vermehrt und mit dem das Kaninchen-βglobin-Gen enthaltenden Vektor transfiziert. 48 Stunden nach der Transfektion wurden 1 bis 2 x 10⁶ Zellen lysiert und die RNA nach der LiCl-Harnstoff-Methode (Auffray, C. et al., Eur. J. Biochem. 107, 303-314 (1980)) extrahiert.

Herstellung einer Kaninchen-β-globin spezifischen Hybridisierungssonde.

Plasmid pBR 322 RßG SV 40 wurde mit Hae III abgebaut und das sich von + 135 bis -75 erstreckende Fragment (Dierks, P. et al., Proc. Natl. Acad. Sci. USA 78, 1411-1415 (1981)) und Van Oyen, A. et al., Science 206, 337-344 (19) wurde auf einem 2%igen Agarosegel isoliert und durch DEAE-Cellulose-Chromatographie weiter gereinigt (Müller, W. et al., J. Mol. Biol. 124, 343-358 (1978)). Das Fragment wurde mit Kälberdarmphosphatase und $^{32}$P-dephosphoriliert und mit γ-$^{32}$P-ATP und $T_4$ Polynukleotidkinase markiert wie beschrieben in (Mantei, N. et al., Gene 10, 1-8 (1980)).

Nuklease $S_1$ Kartierung

Das mit der Kinase behandelte Fragment wurde zusammen mit 10 μg E. coli t-RNA (Boehringer Mannheim) durch äthanol gefällt und in 100 μl 0,4 M/l NaCl, 1 mM/l EDTA, 40 mM/l Pipes-Puffer, pH 6,4 und 80 % Formamid gelöst (Mantei, N. et al., Nature 281, 40-46 (1979). Die cellulare RNA ( = 25 μg) wurde vakuumgetrocknet, in 10 μl Sonde (0,01 pMol, 30.000 cpm) in Puffer, wie oben beschrieben gelöst, denaturiert, in Glaskapillare eingeschmolzen und 16 Stunden bei 48° C hybridisiert. In einem Kontrollversuch wurde die Sonde mit Kaninchen-β-globin m-RNA (Miles) hybridisiert. Die Probe wurde mit 100 μl 0,2 M/l NaCl, 50 mM/l Natriumacetatpuffer pH 4,5, 1 mM/l ZnSO₄ und 0,5 % Glycerin verdünnt und mit 50 Einheiten Nuklease $S_1$ 60 Minuten bei 30° C inkubiert (Weaver, R. et al., Nucl. Acid Res. 7, 1175-1193 (1979)).

Die Proben wurden mit Phenol behandelt, zusammen mit 10 μg E. coli t-RNA durch äthanol gefällt, mit 80 %igem äthanol gewaschen (20 Minuten bei -70° C), vakuumgetrocknet, in 5 μl Färbelösung gelöst (0 05 % Bromphenolblau, 0,05 % Xylolxyanol, 1 mM/l EDTA, 90 % V/V Formamid), 2 Minuten in einem siedenden Wasserbad erhitzt und der Elektrophorese auf 5 % Polyacrylamidgel (89 mM/l Trisbase, 89 mM/l Borsäure, 1 mM/l EDTA und 7 M/l Harnstoff) unterworfen. Das Gel wurde mit Röntgenfilm der Firma Fuji und einem Verstärkerschirm bei -70° C autoradiographiert.

9.2.3 Ergebnisse

Die Größe der geschützten Fragmente konnte durch Vergleich mit Größenmarkern ($^{32}$P-markiertes pBR 322 x Hinf 1 und pBR 322 x Hae III) beurteilt werden. Nicht transfizierte Zellen ergaben keinerlei globinspezifische Hybridisierungssignale (ausgenommen renaturierte Hybridisierungssonde = 210 Bp). Aus den transfizierten immortalisierten menschlichen Epithelzellen extrahierte RNA schützte zwei Fragmente, deren Größe mit 80 bis 90 Bp bestimmt wurde. Derartige Transkripte wurden auch durch Grosveld et al., Nature 295, 120-126 (1982) und Weidle, U. et al., Nature (1981) gefunden und werden dem Vorhandensein einer internen cryptischen Spleißstelle auf dem Kaninchen-β-globin-Gen zugeschrieben. Transcripte, die von der cap-Stelle des Kaninchen-β-globin-Gens stammen, konnten nicht festgestellt werden. In einem Vergleichsversuch wurden HeLa-Zellen mit Plasmid pBR 322 RßG SV 40 transfiziert. Korrekt iniziierte Transcripte (135 Bp geschützt) sowie Transcripte, die auf das Vorhandensein einer internen cryptischen Pleißstelle zurückgeführt werden (Grosveld, G. et al., Nature 295, 120-126 (1982) und Weidle, U. et al., Nature (1983) wurden durch $S_1$-Kartierung festgestellt.

9.2.4 Beurteilung

Die Tatsache, daß Kaninchen-β-globin spezifische Transcripte in immortalisierten menschlichen Epithelzellen feststellbar sind, welche mit einem von SV 40 stammenden eukaryotischen Vektor, der das Kaninchen-βglobin-Gen enthält, transfiziert wurden, zeigt, daß sich diese Zellinie als Wirtsystem für die Expression von wieder eingeführten geklonten Genen eignet.

**Patentansprüche**

1. Verfahren zur Gewinnung von permanent züchtbaren tierischen und humanen Zellinien durch Fusion von normalen tierischen und humanen Zellen mit biologischen Komponenten, welche eine Züchtungsfähigkeit in vitro bewirken, dadurch gekennzeichnet, daß man normale tierische und humane Zellen mit alleine nicht vermehrungsfähigen Zellfragmenten transformierter Zellen fusioniert und in einem Kulturmedium ohne Selektionssubstanzen züchtet.

2. Verfahren nach Anspruch 1, dadurch gekennzeichnet, daß die Fusion in Gegenwart von Polyäthylenglycol oder Sendai-Virus erfolgt.

3. Verfahren nach Anspruch 1 oder 2, dadurch gekennzeichnet, daß man durch Behandlung mit Cytochalasin B erhaltene Karyoplasten oder Cytoplasten von transformierten Zellen verwendet.

4. Verfahren nach Anspruch 1 oder 2, dadurch gekennzeichnet, daß man durch Lyse oder mechanischen Aufschluß von transformierten Zellen erhaltene Zellfragmente, insbesondere Kernfraktionen oder Cytoplasma-Fraktionen verwendet.

5. Verfahren nach Anspruch 1 oder 2, dadurch gekennzeichnet, daß man zur Fusion eine mitochondrienreiche Cytoplasma-Fraktion verwendet.

6. Verfahren nach einem der vorhergehenden Ansprüche, dadurch gekennzeichnet, daß man als transformierte Zellen Myelomzellen, mit Eppstein-Barr-Virus infizierte Zellen oder Aszites-Tumorzellen verwendet.

7. Verwendung einer nach dem Verfahren eines der Ansprüche 1 bis 6 hergestellten permanent züchtbaren menschlichen oder tierischen Zellinie zur Gewinnung von homologen oder/und heterologen Zellprodukten wie monoklonalen Antikörpern, Gerinnungsfaktoren und Lymphokinen oder zur Prüfung von Wirksubstanzen.

8. Abänderung der Verwendung nach Anspruch 7, dadurch gekennzeichnet, daß man aus den Hybridzellen der hergestellten Zellinien die Gene oder die Messenger-RNA für das gewünschte Zellprodukt gewinnt und mit Hilfe eines geeigneten Vektors in einem Mikroorganismus, insbesondere E. coli, transformiert und das Zellprodukt aus dem transformierten Mikroorganismus gewinnt.

**Claims**

1. Process for the obtaining of permanently culturable animal and human cell lines by fusion of normal animal and human cells with biological components which bring about a culturability in vitro, characterised in that one fuses normal animal or human cells with cell fragments of transformed cells which alone are not capable of multiplication and cultures in a culture medium without selection substances.

2. Process according to claim 1, characterised in that the fusion takes place in the presence of polyethylene glycol or Sendai virus.

3. Process according to claim 1 or 2, characterised in that one uses caryoplasts or cytoplasts of transformed cells obtained by treatment with cytochalasin B.

4. Process according to claim 1 or 2, characterised in that one uses cell fragments obtained by lysis or mechanical digestion of transformed cells, especially nuclear fractions or cytoplasma fractions.

5. Process according to claim 1 or 2, characterised in that one uses a mitochondria-rich cytoplasma fraction for the fusion.

6. Process according to one of the preceding claims, characterised in that, as transformed cells, one uses myeloma cells, cells infected with Epstein-Barr virus or ascites tumour cells.

7. Use of a permanently culturable human or animal cell line produced according to the process of one of claims 1 to 6 for the obtaining of homologous and/or heterologous cell products, such as monoclonal antibodies, coagulation factors and lymphokines or for the testing of active substances.

8. Modification of the use according to claim 7, characterised in that, from the hybrid cells of the cell lines produced, one obtains the genes or the messenger RNA for the desired cell product and, with the help of an appropriate vector, transforms into a micro-organism, especially E. coli, and obtains the cell product from the transformed micro-organism.

**Revendications**

1. Procédé d'obtention de lignées cellulaires animales et humaines cultivables de manière permanente par fusion de cellules animales et humaines normales avec des constituants biologiques qui provoquent une aptitude à la culture in vitro, caractérisé en ce qu'on fusionne des cellules animales et humaines normales avec des fragments cellulaires de cellules transformées qui ne peuvent se multiplier seuls, et en ce qu'on les cultive dans un milieu de culture sans substances de sélection.

2. Procédé selon la revendication 1, caractérisé en ce que la fusion s'effectue en présence de polyéthylèneglycol ou de virus Sendai.

3. Procédé selon la revendication 1 ou 2, caractérisé en ce qu'on utilise des karyoplastes ou des cytoplastes de cellules transformées, obtenus par traitement avec la cytochalasine B.

4. Procédé selon la revendication 1 ou 2, caractérisé en ce qu'on utilise des fragments cellulaires obtenus par lyse ou désagrégation mécanique de cellules transformées, en particulier des fractions de noyau ou des fractions de cytoplasme.

5. Procédé selon la revendication 1 ou 2, caractérisé en ce qu'on utilise pour la fusion une fraction de cytoplasme riche en mitochondries.

6. Procédé selon l'une des revendications précédentes, caractérisé en ce qu'on utilise comme cellules transformées des cellules de myélome, des cellules infectées par du virus d'Epstein-Barr ou des cellules de tumeur ascitique.

7. Utilisation d'une lignée cellulaire humaine ou animale cultivable de maniére permanente, préparée selon le procédé d'une des revendications 1 à 6, pour l'obtention de produits cellulaires homologues et/ou hétérologues tels que des anticorps monoclonaux, des facteurs de coagulation et des lymphokines ou pour l'examen de substances actives.

8. Modification de l'utilisation selon la revendication 7, caractérisée en ce que l'on prépare à partir des cellules hybrides des lignées cellulaires préparées, les gènes ou l'ARN messager pour le produit cellulaire souhaité et en ce qu'on les ou le transforme à l'aide d'un vecteur approprié dans un micro-organisme, en particulier E. Coli, et en ce qu'on obtient le produit cellulaire à partir du micro-organisme transformé.